# EUROPEAN PATENT APPLICATION

(11) **EP 1 352 972 A2**
(43) Date of publication of application: **15.10.2003**
(21) Application number: 03006128.7
(22) Date of filing: 18.03.2003
(51) Int. Cl.: C12Q 1/68, G01N 33/50

(54) **Methods of identifying a pharmaceutically active compound for the treatment of a condition caused by altered expression of the insulin receptor**

(30) Priority: 18.03.2002 DE 10211915; 18.03.2002 US 365371 P
(71) Applicant: Jenapharm GmbH & Co. KG, 07745 Jena (DE)
(72) Inventor: Müller, Dieter, 20249 Hamburg (DE); Mukhopadhyay, Amal K., 22523 Hamburg (DE); Wessels, Judith, 20255 Hamburg (DE)
(74) Representative: UEXKÜLL & STOLBERG

(57) **Abstract**

The present invention relates to a method of identifying a pharmaceutically active compound for the treatment of a condition caused by altered expression of the insulin receptor. The invention further relates to a method of diagnosing a condition in a mammal using an *in-vitro* assay. The invention also relates to a method for preparing a pharmaceutical composition for the treatment of a condition caused by altered expression of the insulin receptor.

SEQUENZPROTOKOLL

<110> JENAPHARM GmbH & Co. KG

<120> Method of identifying a pharmaceutically active
      compound for the treatment of a condition caused by
      altered expression of the insulin receptor

<130> P057042

<160> 4

<170> PatentIn Ver. 2.1

<210> 1
<211> 28
<212> DNA
<213> artifical sequence

<220>
<223> PCR oligonucleotide primer

<400> 1
aagaagctta ggcagagtga caagtgac                                    28

<210> 2
<211> 27
<212> DNA
<213> artifical sequence

<220>
<223> PCR oligonucleotide primer

<400> 2
gaagaattca ttcaggaaga ccttcga                                     27

<210> 3
<211> 21
<212> DNA
<213> artifical sequence

<220>
<223> PCR oligonucleotide primer

<400> 3
tcctgcacca ccaactgctt a                                           21

<210> 4
<211> 21
<212> DNA
<213> artifical sequence

<220>
<223> PCR oligonucleotide primer

<400> 4
cgcctgcttc accaccttct t                                           21

## Description

### Field of the invention

The present invention relates to a method of identifying a pharmaceutically active compound for the treatment of a condition caused by altered expression of the insulin receptor. The invention further relates to a method of diagnosing a condition in a mammal using an *in-vitro* assay. The invention also relates to a method for preparing a pharmaceutical composition for the treatment of a condition caused by altered expression of the insulin receptor.

### Background of the invention

Erectile response of penis depends upon its ability to enlarge and reach a sufficient rigidity in response to sexual stimulation enabling a successful penetration and intercourse. The process occurs as a result of the relaxation of smooth muscle cells in the corpus cavernosum and penile arterioles in combination with a simultaneous venoocclusion that causes blood retention in the corpus sinosoides. Non-adrenergic non-cholinergic neurotransmitters and vasoactive substances mediate the local control of the smooth muscle tone. The active smooth muscle relaxation seems to be a pivotal step in a normal erection and it may also be a critical step involved in the erectile dysfunction present in most cases of vascular impotence (1,2,3).

Erectile dysfunction is defined as a consistent inability to obtain or sustain an erection sufficient for intercourse in at least 50% of attempts (4). Presently, erectile dysfunction is determined via patient anamnesis or penile plethysmography.

Age is one of the significant risk factors for erectile dysfunction, which in general is associated with a decreased libido (6). The problems usually found in this condition are a decrease in the duration and degree of penile tumescence. Although erectile dysfunction is often co-indicated with vascular disease such as arteriosclerosis and hypertension, degenerative ultrastructural cellular changes in the corporal tissue are also known to be associated with erectile failure (5,6).

Androgens have long been known to have a major stimulatory effect on several aspects of male sexual behaviour, including penile erection. Androgen treatment of hypogonadal men has been shown to restore sexual interest and activity (7,8). In the past few years several studies have investigated the effects of castration and testosterone replacement on rat erectile response (7). In the penis, androgen depletion leads to smooth muscle apoptosis, a relative increase in connective tissue content and an impaired relaxation potential in the penis (9, 10, 11). In addition, it was shown that androgens are most important for the NO induced erectile activity in rat (12). The main androgen involved in the NO-mediated stimulation of erection is dihydrotestosterone (13,14). It is possible that ageing associated decline in circulatory androgen level may therefore contribute to ageing associated erectile dysfunction, although evidence is not yet available for this.

Another hormone having important relevance for erectile function is insulin which can induce vasodilation caused by a number of potential mechanisms involving either the vacular smooth muscle, vascular endothelium, or both (15-18). Insulin has been shown to have a direct relaxing effect on vascular smooth muscle by hyperpolarisation and decreased calcium influx. It is further suggested that insulin can increase the production of NO by regulating the expression of eNOS in cultured endothelial cells, indicating an activation of NOS via the insulin receptor (19,20). Thus, insulin may modulate vascular tone. Diabetes associated erectile dysfunction resulting mostly from vascular damages have been well investigated (21-23).

The involvement of insulin in the erectile function is well documented as erectile dysfunction is common in diabetes (21,43). Also a possible role of insulin in the nitric oxide controlled system of vasodilatation and vasoconstriction of smooth muscle cells in the rat penis has been proposed by demonstrating a vasodilatory effect of insulin in aortic endothelial cells linking insulin with activation of eNOS (19, 20, 44).

The biological effects of insulin are mediated by a membrane glycoprotein that consists of two extracellular α-subunits, which bind insulin, and two membrane-spanning β-subunits bearing an intrinsic tyrosine kinase activity for transducing the insulin-induced signal inside the cell (24,25). The human insulin receptor is composed of 22 exons which encode the α-subunit (exon 1-11) and the β-subunit (exon 12-22) (26). The mature insulin receptor exists as two isoforms that differ by the presence or absence of 12 amino acids at the carboxy terminus of the α-subunit. These two receptor isoforms are generated by tissue-specific alternative splicing of the 36-basepair exon 11 (27-29). The expression of these two spliced isoforms (Ex11⁺/Ex11⁻) varies depending on tissue type in humans, rats and monkeys (27,28,30,31). Expressed in cultured cells the two insulin receptors exhibit different functional properties. The receptor variant lacking exon 11 (Ex11⁻) shows a higher affinity for insulin (32,33) and a higher internalization rate (34) than the variant comprising exon 11 (Ex11⁺). In contrast, Ex11⁺ isoform has a higher insulin-stimulated tyrosine kinase activity (35).

It has been reported that properties of the receptor are fundamentally affected by splicing (29-31). On one hand, Ex11⁺ shows an enhanced receptor autophosphorylation and receptor substrate phosphorylation suggesting that this form signals more efficiently than Ex11⁻ (35). On the other hand, Ex11⁻ receptor proteins show a higher affinity for insulin and an increased rate of internalisation (32-34).

The effect of ageing on insulin receptor expression in liver, muscle and heart was analyzed by Vidal et al. (41). The authors demonstrated that ageing induces a significant decrease in total insulin receptor mRNA levels in the liver and heart, whereas no alteration was described for the muscle. In all three tissues the proportion of Ex11⁺ mRNA was significantly decreased. A study by Wiersma et (42) revealed that 12 months-old rats were characterized by a significant decrease in the relative expression of Ex11⁺ mRNA in the liver, heart and tibialis muscle (42). On the other hand, they showed that the effect of ageing corresponded to a reduction of the absolute level of Ex11⁺ mRNA in the tissues, without modification of Ex11⁻ mRNA.

Presently, there are only few medicaments for treating erectile dysfunction. However, these medicaments are not efficient in every patient. Moreover, side effects of the compounds make it impossible to use the same on many patients in need of treatment.

Consequently, there is still a need for identifying new compounds which are useful in the treatment of an erectile dysfunction. Until now, however, there is no reliable screening method which would allow evaluation whether or not a compound is effective in treating such conditions.

### Summary of the invention

This problem is solved by a method for identifying a pharmaceutically active compound for the treatment of a condition caused by altered expression of the insulin receptor, comprising the steps of
a) administering a potential pharmaceutically active compound to a mammal; and
b) subsequently *in-vitro* analysing the amount of the Ex11⁺-splice variant and/or the Ex11⁻-splice variant of the insulin receptor in the penile tissue of said mammal; and
c) selecting a compound which leads to an increase of the amount of the Ex11⁺-splice variant and/or a decrease of the amount of the Ex11⁻-variant of the insulin receptor.

It has now suprisingly been found that alteration of the splicing mechanism of the insulin receptor mRNA in the penis tissue during ageing essentially corresponds to those in young subjects which had undergone castration. This offers an improved possibility to identify pharmaceutically active compounds which are useful in the treament of such conditions by their ability to reverse the splicing pattern observed.

In the present application, the term "Ex11⁺-variant" is used to refer to the splice variant of the insulin receptor which comprises exon 11 within its sequence. As used herein, exon 11 defines the exon encoding the C-terminal part of the α-subunit of the insulin receptor protein. In mammals exon 11 embraces a 36 bp fragment encoding 12 amino acids.

The term "Ex11⁻-variant" is used according to the present invention to refer to a splice variant of the insulin receptor which lacks exon 11 within its sequence.

In a preferred embodiment of the invention, the condition caused by altered expression of the insulin receptor is an erectile dysfunction. In the context of the present invention, an erectile dysfunction can be an acute erectile dysfunction, a pathologic condition, ageing-dependent erectile dysfunction, or a predisposition of an erectile dysfunction.

As used herein, erectile dysfunction defines a disturbance of erection, i.e. an inability of the penis to enlarge and reach or sustain suffient rigidity for successful penetration in response to sexual stimulation. The dysfunction may be a primary, i.e. permanent, disfunction, or a secondary dysfunction, i.e. a dysfunction arising spontaneously in distinct situations.

The erectile dysfunction may also be due to organic causes like insufficient blood supply of the tissue due to aterial abnormalities; insufficient insulation of the spongy body, for example by myocytes degeneration; neurogenic disorders, for example mechanical damage on nerves as a result from surgery; or hormone disorders like depletion of testosterone. As used herein, an age-depending erectile dysfunction is an erectile dysfunction the developement of which is associated with the increasing age of a mammal.

A predisposition for an erectile dysfunction as used in the present invention refers to a physiological and genetic condition of a mammal which indicates a risk of developing erectile dysfunction as described above or symptomes associated with erectile dysfunction.

In a further embodiment of the invention, a sample, such as a penile tissue sample, blood or serum is withdrawn from the mammal. The sample is withdrawn and the amount of the Ex11⁺-splice variant and/or the Ex11⁻-splice variant of the insulin receptor is subsequently analyzed. In order to release the receptor protein or a nucleic acid coding therfor from the cells, the cells of the sample can be lysed by means of methods known to a person skilled in the art. These methods include, for example, the addition of lytic enzymes or detergent like SDS to the cell sample as well as the disruption of the cells by sonication or freezing and subsequent thawing. It may also be possible to use blood or serum which directly surround the penile tissue as samples in accordance with the present invention.

The amount of the Ex11⁺- and/or the Ex11⁻-splice variant of the insulin receptor to be analysed can be a relative or an absolute amount. As used herein, the absolute amount of an insulin receptor splice variant refers to the number of the molecules or their weight as detected in a defined sample unit (for example per µg). The number of molecules can be inferred from quantitative detection methods like, for example, RT-PCR or spectrometric measurements known by a person skilled in the relevant art. The sample unit can be defined by standard IUPAC units of measurements like mg or ml.

According to the present invention, the relative amount of an insulin receptor splice variant can be determined by analyzing the amount of molecules relative to the amount of a second molecule from the same or a different sample, which is used as an internal control.

For this purpose, co-detection can be performed under the same conditions as employed for detection of insulin receptor splice variant. When using tissue samples, co-detection can relate to a reference molecule constitutively expressed in the cell. Molecules suitable for co-detection are well-known in the art and comprise, inter alia, the globulin encoding mRNAs. In a particularly preferred embodiment, the relative amount of the Ex11⁺- and/or the Ex11⁻-splice variant of the insulin receptor is determined by comparison of the amount of the Ex11⁻-splice variant with the amount of the Ex11⁺-splice variant of the insulin receptor or with the amount of the enzyme glycerinealdehyde-3-phosphate-dehydrogenase (GAPDH). Preferably both are detected using the same method as used for the insulin splice variant.

In a further embodiment of the invention, *in-vitro* analysing the amount of the Ex11⁺-splice variant and/or the Ex11⁻-splice variant of the insulin receptor can comprise the analysis of an Ex11⁺-splice variant and/or the Ex11⁻-splice variant encoding nucleic acid. Preferably, analysing comprises the determination of the amount of Ex11⁺-splice variant and/or the Ex11⁻-splice variant encoding mRNA. Alternatively, analysing comprises the determination of the amount of the Ex11⁺-splice variant and/or the Ex11⁻-splice variant proteins.

Depending on the nature of molecule to be deteced, analysing can comprise nucleic acid amplification like PCR or RT-PCR, or Blot methods like Southern-, Western- or Northern-Blots employing probes capable of specifically detecting the different splice variants. Analysing can further comprise immuno-binding assays which are known to the skilled person. For example, monoclonal antibodies against the 12 amino acid fragment encoded by exon 11 can be generated and linked to a dye like fluorescein-isothiocyanat to specifically detect the Ex11⁺-variant. Numerous methods for coupling an antibody to a substance suitable for detection are available in the art.

It is, of course, also possible to detect and isolate an insulin receptor splice variant by a procedure allowing isolation of the splice variant and analyzing the relative or absolute amount of the variant by chromatographical or spectroscopical measurements, for instance, by NMR-spectroscopy or HPLC. Methods suitable for such detections are well-known to a person ordinary skilled in the art.

According to the invention, the mammal the potential pharmaceutically active compound is administered to, can be a rodent, e.g. a mouse, a rat, a rabbit, or a guinea pig. Preferably, the rodent is a rat.

In a further embodiment of the invention, the method for identifying a pharmaceutically active compound further comprises the step of comparing the amount of the Ex11⁺-splice variant and/or the Ex11⁻splice variant of the insulin receptor with the amount of the same insulin receptor splice variant determined in the same mammal before administration of the potential pharmaceutically active compound. Comparison of the amounts of a specific insulin receptor splice variant before and after administration of the potentially active compound allows direct evaluation as to whether the compound is capable of increasing and/or decreasing the amount of said splice variant in the affected mammal.

The invention further relates to a method for preparing a pharmaceutical composition for treatment of a condition which is caused by altered expression of the insulin receptor, comprising:
a) identifying a pharmaceutically active compound according to a method of the present invention; and
b) mixing said compound with a pharmaceutically acceptable carrier.

As used herein, the term "pharmaceutically acceptable carrier" includes, for example, solvents, dispersion media, coatings and absorption delaying agents being compatible with pharmaceutical administration. In accordance with the invention, water, saline, propylene glycol, glycerine, Ringer's solution, dextrose solution, solutions containing human serum albumin, as well as fixed oils can be employed as suitable carriers. The use of these carriers is well-known to a person skilled in the art.

According to one embodiment of the invention, the pharmaceutical composition is formulated for parenteral administration. For example, the composition can be formulated for administration by injection. Compositions suitable for injectable use commonly comprise sterile aqueous solutions or dispersions. For intravenous application, suitable carriers include bacteriostatic water, or phosphate buffered saline. The composition can further comprise agents for preventing microbial growth.

Alternatively, the pharmaceutical composition can be formulated for topical administration. These compositions usally comprise penetrants like detergents or other substances which are useful for enabling the active compound to permeate through the barrier to be passed, e.g. the transdermal barrier of the skin. For topical administration, the pharmaceutical compositions can be formulated in form of ointments, salves, gels or creams according to standard methods.

Otherwise, the pharmaceutical composition can be formulated for oral administration. These compositions generally comprise an inert or an edible carrier. The compositions for oral administration can be provided, for example, in the form of tablets, pills, or capsules.

According to a preferred embodiment of the present invention, the pharmaceutical composition is formulated in dosage unit form.

As used herein, "dosage unit form" relates to a physically discrete unit suited as unitary dosage for the mammal to be treated. A unit contains a predetermined quantity of the pharmaceutical composition which is sufficient for generating the desired therapeutic effect. The exact amount of a given active compound which contributes to a unit dosage form clearly depends on the specific compound as well as on the carrier employed.

The invention further relates to method of diagnosing of a condition which is caused by altered expression of the insulin receptor in a mammal comprising *in-vitro* analysing the amount of an insulin receptor splice variant in the penile tissue of said mammal.

In a preferred embodiment of the invention, the insulin receptor splice variant to be determined in the penile tissue is the Ex11⁻-variant and/or the Ex11⁺-variant.

The condition to be diagnosed can be an erectile dysfunction. Preferably, it is an acute, ageing-dependent, or a predisposition for an erectile dysfunction.

The diagnostic method can further comprise withdrawing a sample, such as penile tissue, a blood or serum sample from the mammal and analysing the amount of the insulin receptor splice variant within the sample.

The amount of an insulin receptor splice variant to be analysed can be a relative or an absolute amount. According to a preferred embodiment, the amount of the insulin receptor splice variant to be analysed is a relative amount determined by comparison of the amount of the Ex11⁻-splice variant to the amount of the Ex11⁺-splice variant or to the amount of GAPDH in the sample.

Like for the method described above, analysis can comprise the analysis of a nucleic acid encoding an insulin receptor variant, e.g. an mRNA encoding an insulin receptor variant, as well as the analysis of an insulin receptor protein by means of PCR, RT-PCR, Southern-, Western- or Northern blot, or immuno-binding assays.

According to the invention, the mammal to which diagnosis is applied to can be a mammal, and, more preferably, a human.

According to an alternative embodiment of the invention, the amount of insulin receptor splice variant determined in a mammal is compared with the amount of the insulin receptor splice variant of a control mammal which does not suffer from erectile dysfunction. To detect ageing-dependent erectile dysfunction, a control mammal is advantageously used, which is younger than the mammal which is to be diagnosed. Preferably, the control mammal should be of an age at which substantially a maximum of sexual activity can be predicted with respect to its biological species. As far as diagnosis should be applied to a human, the control mammal should preferably be between 18-25 years old.

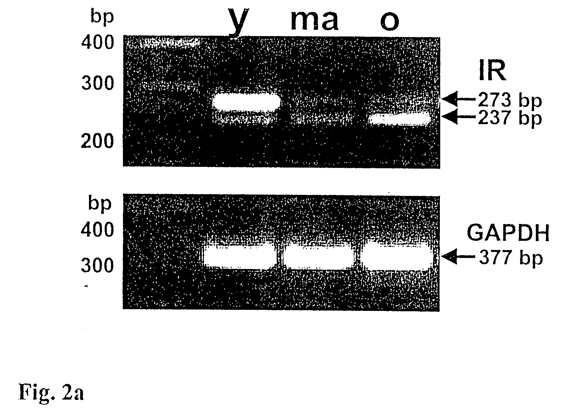

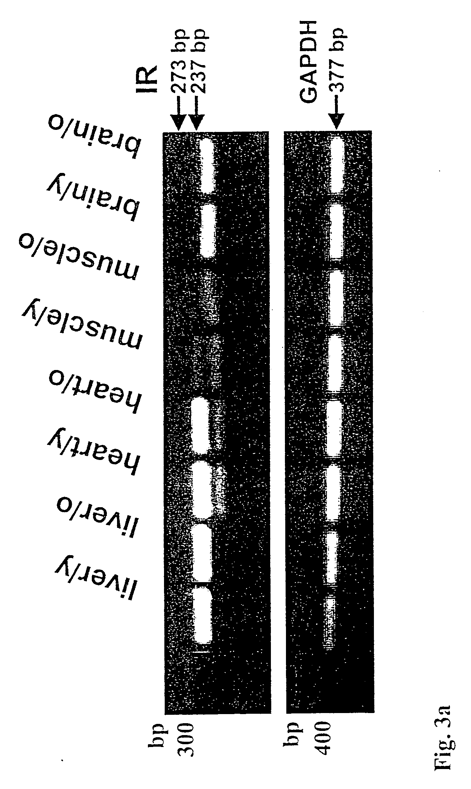

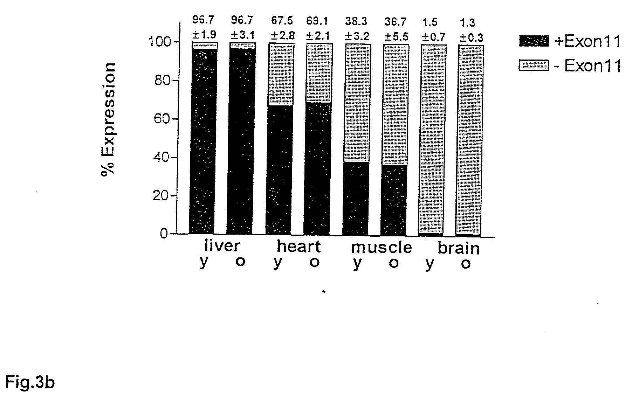

In the course of the present invention, it was shown that the Ex11⁺ variant is predominantly expressed in young rats (3 months old), while the Ex11⁻-variant represents the most abundant splice variant in old rats of 23 months (Fig. 2a). Moreover, it was shown that this alteration is specific for the penis tissue, since it cannot be observed in other tissues like heart, liver or muscle (Fig. 3). Essentially the same expression pattern was found for young castrated rats (3 months).

Since the shift in the relative distribution of splicing variants resulting from ageing and castration is a penis-specific effect, the shift accounts for a lowered insulin sensitivity of penile tissue leading to a local insulin resistance and thereby affecting erectile function. Thus, the specific pattern of the insulin receptor mRNA splicing offers the possibility to diagnose erectile dysfunction in a mammal using an *in-vitro* assay capable of detecting one or both of the splice variants and to identify pharmaceutically active compounds for the treatment of a conditions caused by altered expression of the insulin receptor.

### Brief description of the drawings

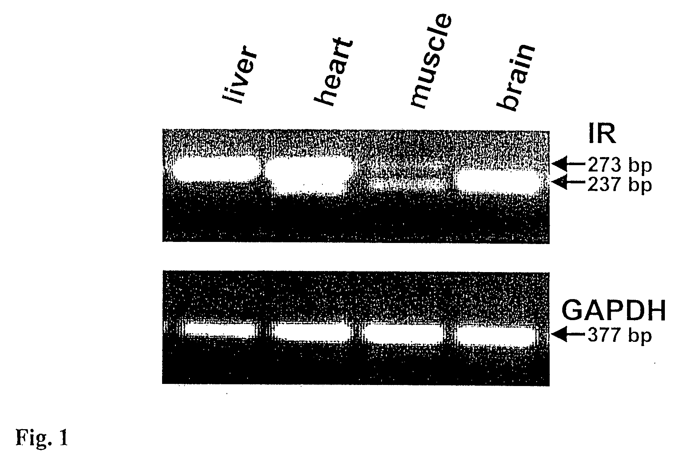

**Fig.1:** Expression of two alternatively spliced IR mRNA transcripts in various tissues of rats as revealed by RT-PCR. Amplification of cDNA was performed by using a primer set flanking the alternatively spliced exon 11. The two predominant amplification products are at 273 bp and/or 237 bp representing the two IR mRNA species with or without exon 11, respectively. GAPDH mRNA was amplified as internal control. Reaction products wre analyzed by electrophoresis in a 2.5% ethidiumbromid stained agarose gel.
**Fig.2a:** Expression of two alternatively spliced IR mRNA transcripts in penile tissue of young (y), middle-aged (ma) and old (o) rats as revealed by RT-PCR. Amplification of cDNA was performed by using a primer set flanking the alternatively spliced exon 11. The two predominant amplification products are at 273 bp and/or 237 bp representing the two IR mRNA species with or without exon 11, respectively. GAPDH mRNA was amplified as internal control. Reaction products were analyzed by electrophoresis in a 2.5% ethidiumbromid stained agarose gel. M = 100 bp DNA ladder.

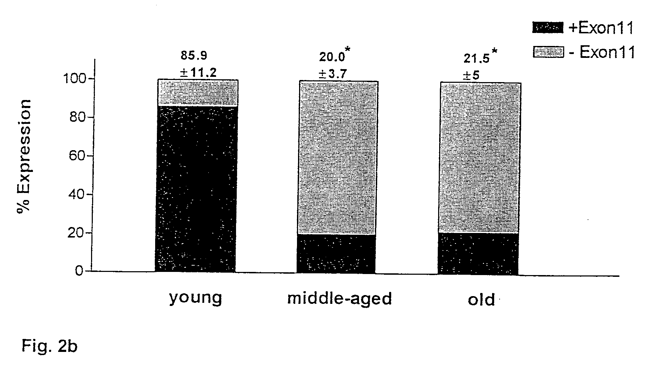

**Fig.2b:** Relative abundance of the two insulin receptor mRNAs spliced variants in penile tissue of young, middle-aged and old rats. Percentage of exon 11 (EX11+)mRNA form is represented by solid portion of bars, with means ± SD of this percentage at left. *p<0,05 vs. young control animals.
**Fig.3a:** Expression of two alternatively spliced IR mRNA transcripts in penile tissues of young (y), and old (o) rats as revealed by RT-PCR. Amplification of cDNA was performed by using a primer set flanking the alternatively spliced exon 11. The two predominant amplification products are at 273 bp and/or 237 bp representing the two IR mRNA species with or without exon 11, respectively. GAPDH mRNA was amplified as internal control. Reaction products were analyzed by electrophoresis in a 2.5% ethidiumbromid stained agarose gel. M = 100 bp DNA ladder.
**Fig.3b:** Relative abundance of the two insulin receptor mRNAs spliced variants in various tissues of young (y), and old (o) rats. Percentage of exon 11 (EX11+)mRNA form is represented by solid portion of bars, with means ± SD of this percentage at top. *p<0,05 vs. young control animals.

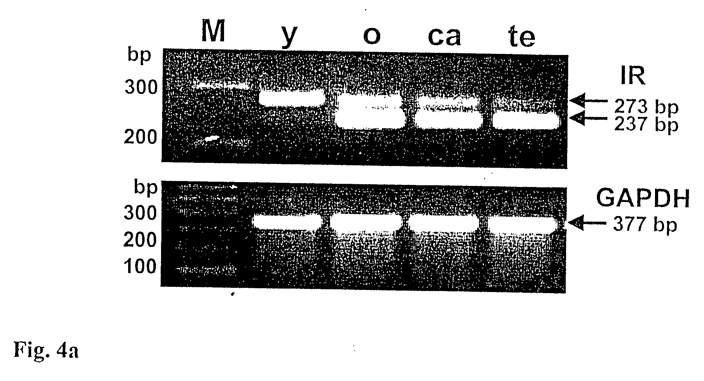

**Fig.4a:** Expression of two alternatively spliced IR mRNA transcripts in penile tissues of young (y), old (o), young castrated (ca) and castrated and testosterone-replaced (te) rats as revealed by RT-PCR. Amplification of cDNA was performed by using a primer set flanking the alternatively spliced exon 11. The two predominant amplification products are at 273 bp and/or 237 bp representing the two IR mRNA species with or without exon 11, respectively. GAPDH mRNA was amplified as internal control. Reaction products were analyzed by electrophoresis in a 2.5% ethidiumbromid stained agarose gel. M = 100 bp DNA ladder.

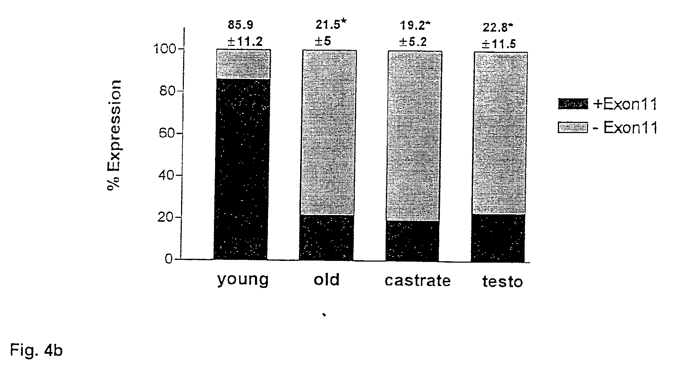

**Fig.4b:** Relative abundance of the two insulin receptor mRNAs spliced variants in penile tissue of young, old, young castrated (castrate) and young castrated but testosterone-replaced (testo) rats. Percentage of exon 11 (EX11+)mRNA form is represented by solid portion of bars, with means ± SD of this percentage at top. *p<0,05 vs. young control animals.

### Examples

### Example 1

### Animals and tissues

Male wistar rats (Charles River, Sulzfeld, Germany) were maintained under standard conditions of housing (12h light, 12h dark cycle, 23°C) with regular laboratory rat food and water ad libitum.

The rats were divided into three groups each containing four animals. Group A consisted of 3 months old, group B consisted of 14 months old and group C included 23 months old rats. Tissue pieces of penis, heart, liver, brain and muscle were collected immediately after the animals were sacrificed. A portion of these tissues were eiter fixed in Bouin's solution for histological evaluation and another portion was frozen in liquid nitrogen for RNA-preparation later on. Blood was collected for the determination of total and free testosterone using radioimmunoassay. The assay was performed according to the instructions of the manufacturer (Testosterone ¹²⁵I RIA Kit, ICN Biomedicalls Inc., USA).

### Example 2

### RNA Preparation

Tissue samples (100-200 mg) were pulverized in liquid nitrogen. Total RNA was isolated using guanidium thocyanate, phenol-chloroform extraction (pecLab, Erlangen, Germany). The RNA was precipitated with isopropanol at -20°C for 2h, washed with 75% ethanol, dissolved in diethylpyrocarbonate water and quantified spectrometrically at 260 nm. The ratio of absorption of all preparations was between 1.8 and 2.0. To ensure that concentration was identical in each sample, the intensity of 28 sRNA was compared on an 1% agarose gelectrophoresis after ethidium bromide staining. Total RNA was stored at -80°C until further use.

### Example 3

### cDNA synthesis and Polymerase chain Reaction amplification

First strand cDNA synthesis was carried out using a commercially available kit (Gibco BRL). Briefly, 4µg of total RNA was primed with 0,5 µg of oligo (dT)₁₂₋₁₈ and incubated 10min at 70°C. For each sample lx first strand puffer, 0,2M DTT, 10mM dNTP's and 200U of Superscript II reverse transcriptase were added. Reverse transcription was performed at 42°C for 50min and stopped by heating for 15 min. at 70°C. The resulting cDNA templates were stored at -20°C or directly used for PCR.

After a denaturation period of 3 min the PCR reaction was followed by 35 cycles of 95°C for 60 sec., 60°C for 60 sec. and 72°C for 60 sec. PCR was performed with 6 µl cDNA, 10 mM of each dNTP, 5U Taq-Polymerase (Biotherm) and 50 pmol of both sense and antisense primers in PCR buffer. The final volume of each reaction was 50µl. Reaction products (15µl) were run on a 2.5% agarose gel. Primers used for PCR were:
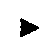 5'-AAGAAGCTTAGGCAGAGTGACAAGTGAC-3' (SEQ ID NO: 1; antisense, complementary to insulin receptor cDNA sequence starting at position 2438 (underlined) and also containing a HindIII restriction site); and
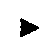 5'-GAAGAATTCATTCAGGAAGACCTTCGA-3' (SEQ ID NO: 2; sense, identical to rat insulin receptor cDNA sequence from position 2181 (underlines) and containing an EcoRI restriction site; 25).

Densitometric analysis of the insulin receptor mRNA splice variant levels was performed using the Image Quant 5.0 software.

For the proportion of the two insulin receptor variant mRNA levels, significance of the difference of the mean values was determined by unpaired student's t-test. The threshold for signigicance was set at p<0.05.

### Example 4

### Alternative splicing of insulin receptor mRNA in diverse tissues of rats

RT-PCR analysis using total RNA from different tissues of rats to determine the relative amounds of alternatively spliced insulin receptor mRNA variants in diverse tissues of young (3 months old) rats was performed according to examples 1-3. Oligonucleotide primers were chosen to flank the corresponding domain of exon 11 splice site of rat insulin receptor representing isoform Ex11⁺ (273 bp) and isoform Ex11⁻ (237 bp). Modifications in the splicing mechanisms were verified by quantitative analysis employing densitometric evaluation. Glycerin aldehyde 3-phosphate-dehydrogenase (GAPDH) mRNA was used as an internal control. PCR-Oligonucleotide primers used for amplification of GAPDH were as follows: primer GAPDH-1: 5'-TCCTGCACCACCAACTGCTTA-3' (SEQ ID NO: 3); and

primer GAPDH-2: 5'-CGCCTGCTTCACCACCTTCTT-3' (SEQ ID NO: 4).

The results of the PCR reaction were examined by agarose gel electrophoresis. As can be seen in figure 1, it was observed that in the liver only longer variants were expressed, which is in agreement with already published data (25,26). In the heart, both variants were found to be expressed, also the ex11⁺ isoform was predominant. In the skeletal muscle both variants were expressed to an equal extent. In the brain, exclusive expression of Ex11⁻ variant was noted (Fig. 1).

### Example 5

### Relative Expression of insulin receptor splice variants in the penis and the effect of ageing

RT-PCR analysis of penis tissue was performed according to examples 1-3. The results are shown in figure 2a. In the penis of young (3 months old) rats, the longer Ex11⁺ isoform was found to be almost exclusively expressed. In contrast, in the penile tissue of old (23 months) rats there was a predominant expression of the shorter Ex11⁻ variant. The distribution of the two splice variants in middle-aged (14 months) animals was similar to the old (Fig.2a). However, the highest expression level of Ex11⁻ insulin receptor could be detected in old rats (Fig. 2a).

Quantitative measurement of insulin receptor Ex11⁺ mRNA in penile tissue of yong versus old rats confirmed that in young penis the most predominant insulin receptor form is Ex11⁺ (85.9-%). In contrast, middle-aged and old rats were characterized by a decrease in the relative expression of Ex11⁺ mRNA in the penis compared to young rats. This switch of the insulin receptor isoform expression in favor of the samller form was significant in both, middle-aged (79.9.%) and old rats (78.4%) as shown in Fig. 2b.

It was of interest, therefore, to investigate if a similar age-dependent variation in the pattern of insulin receptor mRNa expression could be observed in other tissues as well. However, in contrast to the penis, no such age-dependent changes in isoform-specific distribution could be observed in liver, heart, skeletal muscle and brain (Fig. 3a, 3b). Thus, the ageing associated changes in the pattern of insulin receptor mRNA expression was specific to penile tissue.

### Example 6

### Examination of androgen-dependency of ageing-related variation of the pattern of insulin receptor mRNA expression in rat penis by castration and testosterone replacement

Pellets for testosterone-or placebo-implantation were received from Innovative Research of America, Sarasota, Florida, USA. To evaluate the effects of castration and testosterone replacement male wistar rats (3 months, n = 21) were divided into three groups: Group 1, intact (shame operated, given a placebo-pellet by implantation under the dorsal skin, n = 7), group 2, castrated (implantation of placebo-pellet, n = 7) and group 3, castrated, but given a testosterone-implantate of 15 mg (n = 7). 3 weeks after treatment rats were killed and penile tissue as well as blood samples were treated as described in example 1. The experimental study was conducted in accordance with the general federal law on the care and use of laboratory animals.

It was noted that the level of testosterone in the serum of young versus old rats were 4.436 ± 1.1 and 0.57 ± 0.17 ng/ml (mean ± SD, n=4) respectively, pointing to a drop in the level of testosterone in circulation of aged animals. This is in agreement with Zirkins lab (39, 40). In order to discern if the observed ageing related alteration in the expression of two splice variants of insulin receptor mRNA could be due to a declining androgen level in the ageing rats, we have performed orchiedectomy on young rats. One group of castrated rats received androgen releasing subcutanous implant. After castration, the relative weight of the penis in relation to the body weight were dramatically reduced compared to the control animals (77.3 ± 7.3 vs. 46.8 ± 4.3 mg/100g bodyweight). A similar effect was observed with the prostate weight of controled and castrated animals (96.7 ± 30.4 vs. 7.4 ± 2.4; table 1). However, testosterone-replacement induced a reversal of this effect in both organs (table 1). The effect of castration and andorgen-replacement of the insulin receptor mRNA expression in penile tissue of young rats is presented in Fig. 4a. In intact animals, only the longer variant was found to be expressed. Following castration, the appearance of the smaller splice variant was observed, the pattern resembling the old animals. It was noticed that this isoform persisted even in those castrated animals that received androgen supplementation.

A quantative analysis of relative expression of insulin receptor mRNA variants in penis of intact, castrated, castrated and androgen-replacement young rats are shown in figure 4b. In the penis of the intact controls, almost all of the mRNAs encoding the insulin receptor comprised of Ex11⁺ (85.9%) variant. In contrast, in the castrated rats the Ex11⁺ variant accounted for only 19.2% of the total receptor mRNAs, while most (80.8%) of the insulin receptor mRNAs was represented by Ex11⁻ variant. Androgen-supplementation of castrated rats could not alter the relative proportoin of Ex11⁺ to Ex11⁻ being 22.8% and 77.2%, respectively. In both tissues the proportion of Ex11⁺ mRNA was significantly decreased when compared to young control rats.

**Tab.1:**

| Organ weights (mg/100g body weight) of young controle (CON), young castrated (CASTR) and young castrated but testosterone-replaced (TESTO) rats in relation to the body weight. | | | |
|---|---|---|---|
| | CON (mg/100g body weight) | CASTR(mg/100g body weight | TESTO(mg/100g body weight) |
| Penis | 77.3 ± 7.3 | 46.8 ± 4.3 | 78.55 ± 5.54 |
| Prostate | 96.7 ± 30.4 | 7.4 ± 2.4 | 92.37 ± 33.89 |

An ageing-dependent alteration in insultin receptor expression may lead to a situation where signalling mechanisms coupled to insulin rceptors do not funtion optimally, leading to a pathophysiology situation similar to that described for insulin resistance syndrome. In such a syndrome, a diabetes like condition develops although tehre is enough or even increased amaounts of insulin in circulation. Diabetes is associated with an increased incidence of impotence, which is also observed in ageing. It is proposed that altered expression pattern of insulin receptor splice variants in ageing leads to a local condition similar to insulin resistance syndrom,e in the penis. Insulin resistance syndrome has not yet been effectively treated so far. However, compounds like thiazolidinedione insulin sensitisers (see reports by Goldstein BJ, Diabetes Technol. Ther., 3, 267-275 (1999); and Alicia di Rado, www.usc.edu/hsc/info/pr/lv17/721/insulin.html, 2001) have been proposed for treatment of the insulin resistant syndrome. These drugs have many side effects (see www.diabetis-drug.net, 2001) and further research is necessary to develop related compounds that are more effective but have less side effects.

It is conceivable, therefore, that a similar or an improved treatment regimen based on newer compounds or better formulation maybe used to improve the condition of ageing related impotence, the pathophysiology of which appears to be determined by a condition similar to insulin resistant syndrome.
1. Andersson K-E, Wagner G. 1995 The physiology of penile erection. Physiol Rev 75:191-236.
2. Lue T, Dahiya M. 1997 Molecular biology of erectile function and dysfunction. Mol Urol 1:35-48.
3. Udelson D, Nehra A, Hatzichristou D, Azadoi A, Moreland RB, Krane RJ, Saenz de Tejada I, Goldstein I. 1998. Engenesring analysis of penile hemodynamic and structural dynamic relationships. PartI-Clinical implications of penile tissue mechanical properties. Int J Impot Res 10:15-24.
4. Fran E, Kaiser MD. 1999 Erectile dysfunction in the aging man. Med Clin North Am 83:1267-1278.
5. Morgentaler A. 1999 Male impotence. Lancet 354:1713-1718.
6. Monga M. 1999 The aging penis: Erectile dysfunction. Geriatr Nephrol Urol 9:27-37.
7. Mills TM, Dai Y, Stopper VS, Lewis RW. 1999 Androgenic maintenance of the erectile response in in the rat. Steroids 64:605-609.
8. Aversa A, Isidori AM, De Martino MU, Caprio M, Fabbrini E, Rocchietti-March M, Frajese G, Fabbri A. 2000 Androgens and penile erection: evidence for a direct relationship between free testosterone an cavernous vasodilation in men with erectile dysfunction. Clin Endocinol 53:517-522.
9. Baskin LS, Sutherland RS, Di Sandro MJ, Hayward SW, Lipshultz J, Cunha GR. 1997 The effect of testosterone on androgen receptors and human penile growth. J Urol 158:1113-1118.
10. Shabsigh R. 1997 The effects of testosterone on the cavernous tissue and erection. World J Urol 15:21-26.
11. TraishAT, Park K, Kim NN, Moreland RB, Goldstein I. 1999 Effects of castration and androgen replacement on erectile function in a rabbit model. Endocrinology 140:1861-1868.
12. Reilly CM, Zamorano P, Stopper VS, Mills TM. 1997 Androgenic regulation of NO availability in rat penile erection. J Androl 18:110.115.
13. Lugg JA, Rajfer J, Gonzalez-Cadavid NF. 1995 Dihydrotestosterone is the active androgen in the maintenance of nitric oxide-mediated penile erection in the rat. Endocrinology 136:1495-1501.
14. Schirar A, Bonnefond C, Meusnier C, Devinoy E. 1997 Androgens modulate nitric oxide messenger ribonucleic acid expression in neurons of the major pelvic ganglion in the rat. Endocrinology 138:3093-3102.
15. Feener EP, King GL. 1997 Vascular dysfunction in diabetes mellitus. Lancet 350(suppll):SI9-SI13.
16. Scherrer U, Sartorio C. 1997 Insulin as a vascular and sympathoexcitatory hormone: implications for blood pressure regulation, insulin sensitivity and cardiovascular mortality. Circulation 96:4104-4113.
17. Baron AD, Brechtel-Hook C, Johnson A, CroninJ, Learning R, Steinberg HO. 1996 Effect of perfusion rate on the time course of insulin-mediated glucose uptake. Am J Physiol 271:E1067-B1072.
18. Yki-Jarvinen H, Utriainen T. 1998 Insulin-induced vasodilation: physiology or pharmacology? Diabetologia 41:369-379.
19. Zeng G, Quon MJ. 1996 Insulin-stimulated production of nitric oxide is inhibited by wortmannin: direct measurement in vascular endothelial cells. J Clin Invest 98:894-898.
20. Kuboki K, Jiang ZH, Takahara N, Ha SW, Igarashi M, Yamauchi T, Feener EP, Herbert TP, Rhodes CJ, King GL. 2000 Regulation of endothelial constitutive nitric oxide synthase gene expression in endothelial cells in vivo. Circulation 101:676.681.
21. El-Rufaie OEF, Bener A, Abuzeid MSO, Ali TA. 1097 Sexual dysfunction among type II diabetic men: a controlled study. J Psychosom Res 43:605-612.
22. McKendrick JD, Salas E, Dube GP, Murat J, Russell JC, Radomski MW. 1998 Inhibition of nitric oxide generation unmasks vascular dysfunction in insulin-resistant, obese JCR:LA-*cp* rats. B J Urol 124:361-369.
23. Honing MLH, Morrison PJ, Banga JD, Stroes ESG, Rabelink TJ. 1998 Nitric oxide availability in diabetes mellitus. Diabetes Metab Rev 14:241-249.
24. Rosen OM. 1987 After insulin binds. Science 237:1452-1458.
25. White MF, Kahn CR. 1994 The insulin signalling system. J Biol Chem 269: 1-4.
26. Seino S, Seino M, Nishi S, Bell GI. 1989 Structure of the human insulin receptor gene and characterization of its promotor. Proc Natl Acad Sci USA 86:114-118.
27. Seino S, Bell GI. 1989 Alternative splicing of human receptor messenger RNA. Biochem Biophys Res Commun 159:312-316.
28. Goldstein BJ, DudleyAL. 1990 The rat insulin receptor: primary structure and conservation of tissue-specific alternative splicing. Mol Endocrinol 4:235-244.
29. Sugimoto K, Murakawa Y, Zhang W, Xu G, Sima AAF. 2000 Insulin receptor in rat peripheral nerve: its locatization and alternatively spliced isoforms. Diabetes/Metab Res Rev 16:354-363.
30. Moller DE, Yokota A, Caro JF, Flier JS. 1989 Tissue-specific expression of two alternatively spliced insulin receptor mRNAs. Mol Endocrinol 3:1263-1269.
31. Huang Z, Bodkin NL, Ortmeyer HK, Hansen BC, Shuldiner AR. 1994 Hyperinsulinemia is associated with altered insulin receptor mRNAs splicing in muscle of the spontanous obese diabetic rhesus monkey. J Clin Invest 14: 1289-1296.
32. Mostaf L, Grako K, Dull TJ, Coussens L, Ullrich A, McClain DA. 1990 Functionally distinct insulin receptors generated by tissue-specific alternative splicing. EMBO J 9:2409-2413.
33. McClain DA. 1991 Different ligand affinities of the two human insulin receptor splice variants are reflected in parallel changes in sensitivity for insulin action. Mol Endocrinol 5:734-739.
34. Kellerer M, Lammers R, Ermel B, Tippmer S, Vogt B, Obermaier-Kusser B, Ullrich A, Häring HU. 1992 Distinct α-subunit structures of human insulin receptor A and B variants determine differences in tyrosine kinase activities. Biochemistry 13: 4588-4598.
35. Yamaguchi Y, Flier JS, Yokota A, Benecke H, Backer JM, Moller DE. 1991 Functional properties of two naturally occurring isoforms of the human insulin receptor in Chinese hamster ovary cells. Endocrinology 129:2058-2066.
39. Zirkin BR, Chen H, Luo L. 1997 Leydig cell steroidogenesis in aging rats. Exp Gerontol 32:529-537.
40. Zirkin BR, Chen H. 2000 regulation of leydig cell steroidogenic function during aging. Biol Reprod 63:977-981.
41. Vidal H, Auboeuf D, Beylot M, Riou JP. 1995 Regulation of insulin receptor mRNA splicing in rat tissues. Diabetes 44:1196-1201
42. Wiersma MML, Auboeuf D, Nieuwenhuizen-Bakker IM, Radder JK, Riou JP, Vidal H. 1997. Insulin receptor mRNA splicing and altered metabolic control in aged and midly insulin-deficient rats. Am J Physiol 272:E607-E615.
43. Rehman J, Chenven E, Brink P, Peterson B, Walcott B, Wen YW, Melman A, Christ G. 1997 Diminished neurogenic but not pharmacological erections in the 2- to 3-month experimentally diabetic F-344 rat. Am J Physiol (Heart Circ Physiol 41):H1960-1971.
44. Kahn AM, Husid A, Allen JC, Seidel C, Song T. 1997 Insulin acutely inhibits cultured vascular smooth muscle cell contraction by a nitric oxide synthase-independent pathway. Hypertension 30:928-933.

## Claims

1. A method for identifying a pharmaceutically active compound for the treatment of a condition caused by altered expression of the insulin receptor, comprising the steps of
a) administering a potential pharmaceutically active compound to a mammal; and
b) subsequently *in-vitro* analysing the amount of the Ex11⁺-splice variant and/or the Ex11⁻-splice variant of the insulin receptor in the penile tissue of said mammal; and
c) selecting a compound which leads to an increase of the amount of the Ex11⁺-splice variant and/or a decrease of the amount of the Ex11⁻-variant of the insulin receptor.

2. A method according to claim 1, wherein said condition is an erectile dysfunction.

3. A method according to claim 2, wherein said erectile dysfunction is acute, a pathologic condition, ageing-dependent, or a predisposition for an erectile dysfunction.

4. A method according to one of the claims 1 to 3, further comprising withdrawing a sample, such as penile tissue, a blood or serum sample from said mammal and analysing the amount of the Ex11⁺-splice variant and/or the Ex11⁻-splice variant within the sample.

5. A method according to one of the claims 1 to 4, wherein said amount of the Ex11⁺-splice variant and/or the Ex11⁻-splice variant of the insulin receptor is a relative or an absolute amount.

6. A method according to claim 5, wherein said amount of the Ex11⁺-splice variant and/or the Ex11⁻-splice variant of the insulin receptor is a relative amount determined by comparison of the amount of the Ex11⁻-variant to the amount of the Ex11⁺-variant or to the amount of GAPDH in the sample.

7. A method according to one of the claims 1 to 6, wherein *in-vitro* analysing comprises the analysis of a nucleic acid encoding the Ex11⁺-splice variant and/or the Ex11⁻-splice variant of the insulin receptor.

8. A method according to claim 7, wherein *in-vitro* analysing comprises the analysis of a mRNA encoding the Ex11⁺-splice variant and/or the Ex11⁻-splice variant of the insulin receptor.

9. A method according to one of claims 1 to 8, wherein *in-vitro* analysing comprises the analysis of an Ex11⁺-splice variant and/or the Ex11⁻-splice variant protein.

10. A method according to one of claims 7 to 9, wherein said analysis is performed via PCR, RT-PCR, Southern-, Western- or Northern blot, or immuno-binding assays.

11. A method according to one of claims 1 to 10, wherein said mammal is a rodent.

12. A method according to claims 11, wherein said rodent is a rat.

13. A method according to one of the claims 1 to 12, further comprising comparing the amount of the Ex11⁺-splice variant and/or the Ex11⁻-splice variant of the insulin receptor with the amount of the same splice variant as determined in the same mammal before administration of the potential pharmaceutical compound.

14. A method for preparing a pharmaceutical composition for the treatment of a condition caused by altered expression of the insulin receptor, comprising:
a) identifying a pharmaceutically active compound according to one of the claims 1 to 13; and
b) formulating said compound with a pharmaceutically acceptable carrier.

15. A method according to claim 14, wherein said pharmaceutical composition is formulated for parenteral administration.

16. A method according to claim 15, wherein said pharmaceutical composition is formulated for administration by injection.

17. A method according to claim 15, wherein said pharmaceutical composition is formulated for topical administration.

18. A method according to claim 14, wherein said pharmaceutical composition is formulated for oral administration.

19. A method according to one of claims 14 to 18, wherein said pharmaceutical composition is formulated in dosage unit form.

20. A method of diagnosing of a condition caused by altered expression of the insulin receptor in a mammal comprising *in-vitro* analysing the amount of an insulin receptor splice variant in the penile tissue of said mammal.

21. A method according to claim 20, wherein said insulin receptor splice variant is the Ex11⁻-variant and/or the Ex11⁺-variant.

22. A method according to one of the claims 20 to 21, wherein said condition is an erectile dysfunction.

23. A method according to claim 22, wherein said erectile dysfunction is acute, ageing-dependent, or a predisposition for an erectile dysfunction.

24. A method according to claim 20 to 23, further comprising withdrawing a sample, such as penile tissue, a blood or serum sample from the mammal and analysing the amount of an insulin receptor splice variant within the sample.

25. A method according to one of claims 20 to 24, wherein said amount of an insulin receptor splice variant to be analysed is a relative or an absolute amount.

26. A method according to claim 25, wherein said amount of an insulin receptor splice variant to be analysed is a relative amount determined by comparison of the amount of the Ex11⁻-splice variant to the amount of the Ex11⁺-splice variant or to the amount of GAPDH in the sample.

27. A method according to one of the claims 20 to 26, wherein *in-vitro* analysing comprises the analysis of a nucleic acid encoding an insulin receptor variant.

28. A method according to claim 27, wherein *in-vitro* analysing comprises the analysis of an mRNA encoding an insulin receptor variant.

29. A method according to one of claims 20 to 28, wherein *in-vitro* analysing comprises the analysis of an insulin receptor protein.

30. A method according to one of claims 27 to 29, wherein said analysis is performed via PCR, RT-PCR, Southern-, Western- or Northern blot, or immuno-binding assays.

31. A method according to claim 30, wherein said mammal is a human.

32. A method according to one of the claims 20 to 31, further comprising comparing the amount of the insulin receptor splice variant with the amount of insulin receptor splice variant of a control mammal not suffering from said condition.
